(19) Europäisches Patentamt
European Patent Office
Office européen des brevets

(11) Veröffentlichungsnummer: **0 663 385 A2**

# (12) EUROPÄISCHE PATENTANMELDUNG

(21) Anmeldenummer: **94118108.3**

(22) Anmeldetag: **17.11.94**

(51) Int. Cl.[6]: **C07C 63/313**, C08G 59/58, C09D 163/00

(30) Priorität: **14.01.94 DE 4400928**
**03.02.94 DE 4403225**

(43) Veröffentlichungstag der Anmeldung:
**19.07.95 Patentblatt 95/29**

(84) Benannte Vertragsstaaten:
**AT BE CH DE ES FR GB IT LI NL**

(71) Anmelder: **HÜLS AKTIENGESELLSCHAFT**
**D-45764 Marl (DE)**

(72) Erfinder: **Gras, Dr. Rainer**
**Im Ostholz 49a**
**D-44879 Bochum (DE)**
Erfinder: **Wolf, Dr. Elmar**
**Stauffenbergstrasse 7**
**D-45661 Recklinghausen (DE)**

## (54) Salze der Pyromellitsäure, ein Verfahren zu ihrer Herstellung sowie deren Verwendung.

(57) Die Erfindung betrifft Salze der Pyromellitsäure, wobei ihre Amin-Komponente folgende Zusammensetzung hat:

A) 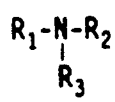 oder B) 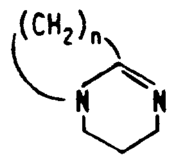

wobei $R_1$, $R_2$, $R_3$ gleiche oder verschiedene aliphatische, cycloaliphatische, araliphatische, aromatische Kohlenwasserstoffreste mit 1 - 20 C-Atomen, wobei in der C-Kette eine oder mehrere $CH_2$-Gruppen durch O-Atome, durch $NR_4$ mit $R_4$ = $C_{1-6}$-Alkyl, durch CH-OH-Gruppen, und/oder eine oder mehrere endständige Methylgruppen durch dialkylsubstituierte Aminogruppen mit 1 bis 6 Kohlenstoffatomen, ersetzt sein können und $R_1$ und $R_2$ einen gemeinsamen Ring bilden können, in dem eine $CH_2$-Gruppe durch ein O-Atom oder durch eine $NR_4$-Gruppe ersetzt sein kann, und $R_1$ = $R_2$ = $R_3$ = $-CH_2-CH_2-$über ein gemeinsames N-Atom gebunden sind, und n : 3 - 11 bedeuten.

Die Erfindung betrifft weiterhin die Herstellung der Salze sowie ihre Verwendung für Epoxid- oder Hybrid-Pulverbeschichtungen.

EP 0 663 385 A2

Die Erfindung betrifft neue Salze der Pyromellitsäure, ein Verfahren zu ihrer Herstellung sowie deren Verwendung zur Herstellung von matten Epoxid- sowie Hybrid-Pulverbeschichtungen.

In der DE-OS 23 24 696 wird ein Verfahren zur Herstellung von matten Überzügen beschrieben, indem man Epoxidharze mit Salzen aus Pyromellitsäure und cyclischen Amidinen härtet. Unserer Kenntnis nach finden sich in der Literatur keine Hinweise, daß noch andere Salze der Pyromellitsäure zur Herstellung von matten EP-Pulverbeschichtungen geeignet sind.

So war es Aufgabe der Erfindung, nach weiteren, für die Matthärtung geeigneten, Salzen zu suchen.

Es wurde überraschend gefunden, daß man auch matte EP- und Hybridpulverbeschichtungen mit Salzen der Pyromellitsäure und den nachfolgend beschriebenen Aminen erhalten kann.

Gegenstand der vorliegenden Erfindung sind somit Salze der Pyromellitsäure, welche dadurch gekennzeichnet sind, daß ihre Aminkomponente folgende Zusammensetzung hat:

A) 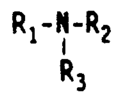 oder B) 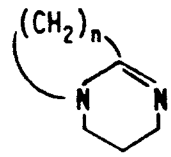

wobei $R_1$, $R_2$, $R_3$ gleiche oder verschiedene aliphatische, cycloaliphatische, araliphatische, aromatische Kohlenwasserstoffreste mit 1 - 20 C-Atomen, wobei in der C-Kette eine oder mehrere $CH_2$-Gruppen durch O-Atome, durch $NR_4$ mit $R_4$ = $C_{1-6}$-Alkyl, durch CH-OH-Gruppen, und/oder eine oder mehrere endständige Methylgruppen durch dialkylsubstituierte Aminogruppen mit 1 bis 6 Kohlenstoffatomen, ersetzt sein können und $R_1$ und $R_2$ einen gemeinsamen Ring bilden können, in dem eine $CH_2$-Gruppe durch ein O-Atom oder durch eine $NR_4$-Gruppe ersetzt sein kann, und $R_1$ = $R_2$ = $R_3$ =$-CH_2-CH_2-$ über ein gemeinsames N-Atom gebunden sind, und n : 3 - 11 bedeuten.

Zur Herstellung der erfindungsgemäßen Salze der Pyromellitsäure eignen sich N-Verbindungen, die zur Salzbildung befähigt sind, wie z. B. N.N-Dimethylcyclohexylamin, N.N-Dimethylanilin, N-Methylmorpholin, N.N'-dimethylpiperazin, 2.2.6.6-Tetramethyl-4-dimethylaminopiperidin, N.N-Di-methyloctadecylamin, N.N-Dimethyl-hexadecylamin, 1.8-Diazabicyclo-[5.4.0]undec-7-en, N.N.N'N'-Tetramethylhexamethylendiamin, N.N.N'.N'.N''-Pentamethyl-diethylentriamin.

Der basische N-Gehalt der erfindungsgemäßen Salze beträgt 1 - 10 mmol/g, der Carboxylgruppengehalt 3 - 13 mmol/g.

Bei den erfindungsgemäßen Verbindungen handelt es sich um farblose bis z. T. intensiv gelb gefärbte Substanzen mit Schmelzpunkten von 140 °C bis ca. 250 °C. Ihre Herstellung erfolgt in bekannter Weise, wobei zu der in Wasser oder Ethanol gelösten Pyromellitsäure in der Siedehitze die Aminkomponente portionsweise zugegeben wird.

Nach beendeter Aminzugabe wird noch ca. eine Stunde weiter erhitzt. Anschließend wird auf Raumtemperatur abgekühlt. Der gebildete Niederschlag wird abfiltriert und im Vakuumtrockenschrank bei 60 bis 80 °C getrocknet.

Ein weiterer Gegenstand der Erfindung ist somit ein Verfahren zur Herstellung von Salzen der Pyromellitsäure nach Anspruch 2, welches dadurch gekennzeichnet ist, daß 1 mol Pyromellitsäure mit 0,5 - 2 mol Amin A) oder B) in $H_2O$ bzw. Ethanol bei 50 - 100 °C umgesetzt und nach beendeter Reaktion das Reaktionsprodukt vom Lösungsmittel abgetrennt wird.

Ferner ist Gegenstand der Erfindung die Verwendung der Salze der Pyromellitsäure nach Anspruch 1 zur Herstellung von matten Epoxid- oder Hybrid-Pulverbeschichtungen, wobei das EP-Harz neben den gegebenenfalls vorhandenen COOH-enthaltenden Polyestern 1 - 12 Gew.-%, vorzugsweise 2 - 7 Gew.-%, insbesondere 3 - 5,5 Gew.-%, der Salze enthält.

Die Gew.-%-Angaben beziehen sich dabei auf die Summe von Epoxid und gegebenenfalls vorhandenen COOH-haltigen Polyestern und gegebenenfalls zusätzlich vorhandenen blockierten Polyisocyanaten, z. B. mit $\epsilon$-Caprolactam blockierte Polyisocyanate und/oder blockierungsmittelfreie Polyisocyanate.

Die verwendeten Polyepoxide sind feste, harzartige Stoffe, die im Bereich 60 - 150 °C, vorzugsweise 70 - 110 °C, schmelzen und die im Durchschnitt mehr als eine 1.2-Epoxidgruppe pro Molekül enthalten. Im Prinzip kommen alle Verbindungen infrage, die mehr als eine 1.2-Epoxidgruppe pro Molekül enthalten; bevorzugt werden allerdings handelsübliche EP-Harze, wie sie durch Umsetzung von Bisphenol A und Epichlorhydrin erhalten werden, mit einem EP-Äquivalentgewicht zwischen 400 - 3000, bevorzugt 800 - 1000, eingesetzt.

Bei den carboxylgruppenhaltigen Polymeren handelt es sich um Polyesterpolycarbonsäuren, die aus Polyolen und Polycarbonsäuren bzw. deren Derivaten hergestellt werden. Der Schmelzbereich dieser sauren Polyester liegt in einem Bereich von 60 - 160 °C, vorzugsweise 80 - 120 °C; ihre Säurezahl variiert von 10 - 150 mg KOH/g, vorzugsweise 30 - 60 mg KOH/g. Die OH-Zahlen sollen unter 10 mg KOH/g liegen.

Für die Herstellung der erfindungsgemäß zu verwendenden Polyesterpolycarbonsäurenwerden Polycarbonsäuren, wie z. B. Oxal-, Adipin-, 2.2.4(2.4.4)-Trimethyladipin-, Azelain-, Sebacin-, Decandicarbon-, Dodecandicarbon-, Fumar-, Phthal-, Isophthal-, Terephthal-, Trimellit-, Pyromellitsäure eingesetzt. Als Polyole für die sauren Polyester werden folgende verwendet: Ethylenglykol, 1.2- und 1.3-Propandiol, 1.2-, 1.3-, 1.4- und 2.3-Butandiol, 1.5-Pentandiol, 3-Methyl-1.5-pentandiol, Neopentylglykol, 1.6-Hexandiol, 1.12-Dodecandiol, 2.2.4(2.4.4)-Trimethyl-1.6-hexandiol, Trimethylolpropan, Glyzerin, Pentaerythrit, 1.4-Bishydroxymethyl-cyclohexan, Cyclohexan-1.4-diol, Diethylenglykol, Triethylenglykol sowie Dipropylenglykol. Selbstverständlich können auch Hydroxylgruppen enthaltende Polyester, die nach bekannten Verfahren aus Polycarbonsäuren und Polyolen hergestellt werden, mit Polycarbonsäuren und/oder Polycarbonsäureanhydriden zu den Polyesterpolycarbonsäuren umgesetzt werden.

Die Mengen der einzelnen Pulverlackbindemittelkomponenten können weitgehend variiert werden.

Im Falle der ausschließlichen Verwendung der handelsüblichen EP-Harze auf Bisphenol A-Basis (+Epichlorhydrin) beträgt die Härterkonzentration 3 - 12 Gew.-%. Im Falle der Verwendung von Gemischen aus Epoxidharzen des Typs Diglycidylester des Bisphenol A und Carboxylgruppen enthaltender Polyester richtet sich das Mengenverhältnis nach der Säurezahl des Carboxylpolyesters. So wird z. B. üblicherweise bei einer Säurezahl 30 - 50 mg KOH/g das Gewichtsverhältnis EP-Harz/Carboxylpolyester 60 : 40 bis 80 : 20, vorzugsweise 70 : 30 betragen. Die Konzentration der erfindungsgemäßen Salze beträgt in diesen EP-Harz/Carboxylpolyester-Gemischen 2 - 10 Gew.-%. Zur Herstellung des Pulverlacks werden die Bindemittel zusammen mit dem Verlaufmittel, Pigment und/oder Füllstoff und den UV- wie Oxidationsstabilisatoren zunächst gemischt und in einem Extruder bei ca. 80 - 130 °C homogenisiert. Das Extrudat wird nach Abkühlung auf Raumtemperatur zu einem Pulverlack gemahlen, wobei die mittlere Teilchengröße ca. 40 - 80 µm, vorzugsweise 50 µm betragen sollte.

Das Auftragen der so hergestellten Pulverlacke auf geeignete Substrate kann nach den bekannten Verfahren, wie z. B. durch elektrostatisches Pulversprühen, Wirbelsintern erfolgen. Nach dem Auftragen des Pulverlacks nach einem der genannten Verfahren werden die beschichteten Substrate zur Aushärtung auf Temperaturen von 150 - 220 °C innerhalb von 30 - 8 min erhitzt. Die so hergestellten Lackfilme zeichnen sich durch sehr guten Verlauf und eine hervorragende Lösemittelbeständigkeit und eine matte Oberfläche aus, wobei der Glanzgrad in einem weiten Bereich beliebig einstellbar ist.

**I. Herstellung der erfindungsgemäßen Salze**

Allgemeine Herstellungsvorschriften

Die in der folgenden Tabelle aufgeführten Salze der Pyromellitsäure wurden in folgender Weise hergestellt:

a) Zu der in Ethanol gelösten Pyromellitsäure wurde das Amin portionsweise zugegeben. Nach beendeter Aminzugabe wurde noch ca. 1 h weiter erhitzt (bei 60 °C). Danach wurde auf Raumtemperatur abgekühlt. Der gebildete Niederschlag wurde abfiltriert und zur vollständigen Entfernung des Ethanols im Vakuumtrockenschrank bei 60 °C getrocknet.

b) Zu der in Wasser gelösten Pyromellitsäure wurde das Amin zugetropft. Nach beendeter Aminzugabe wurde noch ca. 1 h weiter erhitzt (bei 60 - 80 °C) und anschließend das Wasser abdestilliert. Zum vollständigen Entfernen des Wassers wurde im Vakuumtrockenschrank bei 80 °C getrocknet.

| Beisp. | Pyromellitsäure (mol) | (mol) | Amin | Schmelzbereich (°C) | $NH_2$ ($\frac{mmol}{g}$) | COOH ($\frac{mmol}{g}$) |
|---|---|---|---|---|---|---|
| 1 | 1 | 1 | 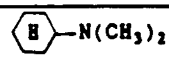 | 188-197 | 2,601 | 10,263 |
| 2 | 1 | 2 | 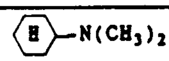 | 192-201 | 3,865 | 7,923 |
| 3 | 1 | 1 | $(CH_3)_2N-C_{16}H_{33}$ | 195-215 | 1,892 | 7,557 |
| 4 | 1 | 1 | 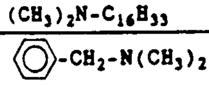 | 184-189 | 2,5112 | 10,051 |
| 5 | 1 | 1 | $(CH_3)_2-N-C_{18}H_{37}$ | 191-206 | 1,801 | 7,196 |
| 6 | 1 | 1 | $(CH_3-CH-CH_2)_3N$<br>OH | 165-179 | 2,210 | 8,814 |
| 7 | 1 | 1 | $(CH_3)_2-N-(CH_2)_2-OH$ | 176-192 | 2,901 | 11,592 |
| 8 | 1 | 1 | 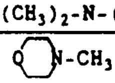 | 225-229 | 2,917 | 11,166 |
| 9 | 1 | 2 | 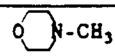 | 190-196 | 4,349 | 8,800 |
| 10 | 1 | 1 | 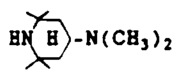 | 226-237 | 4,466 | 9,032 |
| 11 | 1 | 1 | 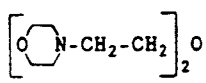 | 120-129 | 9,105 | 9,200 |
| 12 | 1 | 1 | 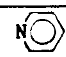 | 231-238 | 2,765 | 11,520 |
| 13 | 1 | 1 | 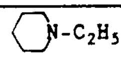 | 223-230 | 2,719 | 10,678 |
| 14 | 1 | 2 | 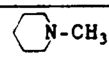 | 188-195 | 2,825 | 11,002 |
| 15 | 1 | 0,5 | $CH_3$<br>$>N-(CH_2)_2-N-(CH_2)_2-N<$ | 176-183 | 3,348 | 11,667 |
| 16 | 1 | 1 | $CH_3$<br>$>N-(CH_2)_2-N-(CH_2)_2-N<$ | 180-185 | 4,562 | 9,301 |
| 17 | 1 | 0,5 | $>N-(CH_2)_6-N<$ | 217-222 | 2,870 | 11,664 |
| 18 | 1 | 1 | $>N-(CH_2)_6-N<$ | 225-234 | 4,544 | 9,373 |
| 19 | 1 | 0,5 | $>N-(CH_2)_2-N<$ | 227-234 | 2,915 | 12,431 |
| 20 | 1 | 1 | $>N-(CH_2)_2-N<$ | 236-243 | 4,960 | 10,633 |
| 21 | 1 | 1 | 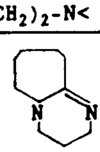 | 210-218 | 2,393 | 8,751 |
| 22 | 1 | 2 | • | 160-180 | 3,304 | 7,018 |

| Beisp. | Pyromellitsäure (mol) | (mol) Amin | Schmelzbereich (°C) | $NH_2$ ($\frac{mmol}{g}$) | COOH ($\frac{mmol}{g}$) |
|---|---|---|---|---|---|
| 23 | 1 | 1 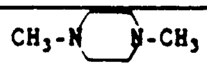 | 258-270 | 2,690 | 10,712 |
| 24 | 1 | 1 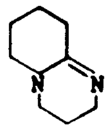 | 206-215 | 2,461 | 9,965 |
| 25 | 1 | 1 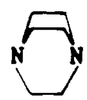 | > 300 | 2,621 | 10,781 |
| 26 | 1 | 0,5 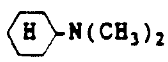 0,5 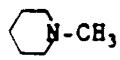 | 180-190 | 2,631 | 10,313 |
| 27 | 1 | 0,5 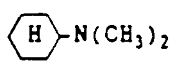 0,5 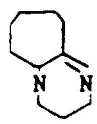 | 186-195 | 2,456 | 9,431 |
| 28 | 1 | 0,5 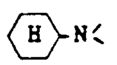 0,5 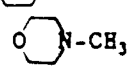 | 178-186 | 2,753 | 10,715 |

**II. Epoxidharze**

In den Anwendungsbeispielen wurden als Epoxidharzverbindungen solche auf Bisphenol A-Basis eingesetzt. Sie sind durch folgende Kenndaten charakterisiert:

| Kenndaten / Beispiel | II. 1 | II.2 |
|---|---|---|
| Äquivalentgewicht | 900 - 1 000 | 1 700 - 2 000 |
| Epoxidwert | 0,1 - 0,111 | 0,05 - 0,058 |
| Hydroxylwert | 0,34 | 0,36 |
| Schmelzbereich °C | 96 - 104 | 125 - 132 |

**III. Epoxidharz-Pulverlacke**

Zur Herstellung der Pulverlacke wurden die gemahlenen Produkte, Härter, Epoxidharz und Verlaufmittel-

Masterbatch[1] mit dem Weißpigment ($TiO_2$) in einem Kollergang innig vermischt und anschließend im Extruder bei 90 bis 120 °C homogenisiert. Nach dem Erkalten wurde das Extrudat gebrochen und in einer Stiftmühle auf eine Korngröße < 100 µm gemahlen. Das so hergestellte Pulver wurde mit einer elektrostatischen Pulverspritzanlage bei 60 kV auf entfettete, gegebenenfalls vorbehandelte Stahlbleche appliziert und in einem Labor-Umlufttrockenschrank eingebrannt.

Die Abkürzungen in den folgenden Tabellen bedeuten:

SD = Schichtstärke (µm)
ET = Tiefung n. Erichsen (mm) (DIN 53 156)
GS = Gitterschnittprüfung (DIN 53 151)
GG 60 °∡ = Glanz n. Gardner (ASTM-D 523)
Imp. rev. = Impact reverse (g•m)

MEK (Methylethylketon)-Festigkeit:
Anzahl Schübe mit MEK-getränktem Wattebausch unter 1 kg-Belastung bis zum Angriff der Lackoberfläche.

Beispiel 1

Nach dem beschriebenen Verfahren wurde der Pulverlack mit folgender Rezeptur hergestellt, appliziert und zwischen 200 °C und 180 °C eingebrannt.

500,0 Gew.-T. Epoxid gemäß II.1
50,0 Gew.-T. Vernetzer gemaß I.1
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60 °∡ | MEK-Festigkeit |
| 15 | 200 | 60 - 65 | 100 | <115,2 | 3,0 - 3,5 | 0 | 10 | 10 |
| 20 | | 70 - 75 | 100 | 115,2 | 5,2 - 6,5 | 0 | 10 | 12 |
| 25 | | 70 - 80 | 100 | 230,4 | 5,0 - 5,5 | 0 | 10 | 16 |
| 30 | 180 | 50 - 60 | 100 | 115,2 | 4,7 - 4,9 | 0 | 9 | 10 |

Beispiel 2

Nach dem beschriebenen Verfahren wurde der Pulverlack mit folgender Rezeptur hergestellt, appliziert und zwischen 170 °C und 220 °C eingebrannt.

480,0 Gew.-T. Epoxid gemäß II.1
70,0 Gew.-T. Vernetzer gemäß I.1
400,0 Gew.-T. Weißpigment ($TiO_2$)
50,0 Gew.-T. Verlaufmittel-Masterbatch

[1] Verlaufmittel Masterbatch
10 Gew.-% Verlaufmittel auf Basis von polymeren Butylacrylaten werden mit den Epoxidharzen in der Schmelze homogenisiert und nach dem Erstarren zerkleinert.

| Einbrennbedingungen | | Mechanische Kenndaten | | | | | | Lösungsmittelresistenz |
| --- | --- | --- | --- | --- | --- | --- | --- | --- |
| Zeit min/Temp. °C | | SD | HB | Imp. rev. | ET | GS | GG 60°∡ | MEK-Festigkeit |
| 6 | 220 | 60 - 80 | 111 | 230,4 | 5,5 - 6,0 | 0 | 18 | 70 |
| 12 | 200 | 70 - 90 | 111 | 345,6 | 5,3 - 5,9 | 0 | 20 | 80 |
| 15 | | 70 - 80 | 111 | 460,8 | 6,5 - 6,7 | 0 | 18 | 90 |
| 20 | | 60 - 80 | 111 | 576 | 6,5 - 7,6 | 0 | 18 | 100 |
| 15 | 180 | 70 | 100 | 115,2 | 3,4 - 4,2 | 0 | 19 | 60 |
| 20 | | 65 - 90 | 111 | 115,2 | 4,5 - 4,9 | 0 | 20 | 70 |
| 25 | 170 | 70 - 80 | 100 | 115,2 | 4,1 - 4,4 | 0 | 18 | 30 |

Beispiel 3

Nach dem beschriebenen Verfahren wurde der Pulverlack mit folgender Rezeptur hergestellt, appliziert und zwischen 200 °C und 180 °C eingebrannt.

470,0 Gew.-T. Epoxid II.1
80,0 Gew.-T. Vernetzer gemäß I.1
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
| --- | --- | --- | --- | --- | --- | --- | --- | --- |
| Zeit min/Temp. °C | | SD | HB | Imp. rev. | ET | GS | GG 60°∡ | MEK-Festigkeit |
| 10 | 200 | 60 | 100 | 460,8 | 4,6 - 4,9 | 0 | 21 | 70 |
| 12 | | 60 - 80 | 100 | 460,8 | 5,1 - 5,8 | 0 | 20 | 80 |
| 15 | | 50 - 60 | 111 | 576 | 6,0 - 6,3 | 0 | 19 | 100 |
| 15 | 180 | 70 | 111 | 230,4 | 4,4 - 4,6 | 0 | 22 | 60 |
| 20 | | 70 - 80 | 111 | 345,6 | 4,7 - 5,3 | 0 | 21 | 70 |

Beispiel 4

Nach dem beschriebenen Verfahren wurde der Pulverlack mit folgender Rezeptur hergestellt, appliziert und zwischen 200 °C und 180 °C eingebrannt.

495,0 Gew.-T. Epoxid gemäß II.1
55,0 Gew.-T. Vernetzer gemäß I.3
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
| --- | --- | --- | --- | --- | --- | --- | --- | --- |
| Zeit min/Temp. °C | | SD | HB | Imp. rev. | ET | GS | GG 60°∡ | MEK-Festigkeit |
| 20 | 200 | 60 - 80 | 100 | <115,2 | 2,7 - 4,1 | 0 | 21 | 8 |
| 25 | | 50 - 65 | 100 | 115,2 | 4,2 - 4,8 | 0 | 19 | 10 |
| 30 | | 70 - 80 | 100 | 115,2 | 5,6 - 6,2 | 0 | 19 | 10 |
| 30 | 180 | 70 - 80 | 100 | <115,2 | 3,8 - 4,5 | 0 | 26 | 8 |

Beispiel 5

Nach dem beschriebenen Verfahren wurde der Pulverlack mit folgender Rezeptur hergestellt, appliziert und zwischen 180 °C und 200 °C eingebrannt.

485,0 Gew.-T. Epoxid gemäß II.1
65,0 Gew.-T. Vernetzer gemäß I.3
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60 °∢ | MEK-Festigkeit |
| 20 | 200 | 70 - 80 | 100 | 115,2 | 5,6 - 5,7 | 0 | 18 | 10 |
| 25 | 200 | 50 - 60 | 100 | 345,6 | 6,1 - 6,7 | 0 | 20 | 12 |
| 30 | 200 | 70 - 80 | 100 | 345,6 | 6,2 | 0 | 20 | 12 |
| 25 | 180 | 70 - 80 | 100 | <115,2 | 2,6 - 3,5 | 0 | 20 | 8 |
| 30 | 180 | 65 - 80 | 100 | 115,2 | 3,3 - 3,9 | 0 | 21 | 10 |

Beispiel 6

Nach dem beschriebenen Verfahren wurde der Pulverlack mit folgender Rezeptur hergestellt, appliziert und zwischen 200 °C und 180 °C eingebrannt.

490,0 Gew.-T. Epoxid gemäß II.1
60,0 Gew.-T. Vernetzer gemäß I.4
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60 °∢ | MEK-Festigkeit |
| 15 | 200 | 60 - 70 | 111 | 115,2 | 5,3 - 5,9 | 0 | 17 | 10 |
| 20 | 200 | 60 | 111 | 115,2 | 6,0 - 6,4 | 0 | 19 | 14 |
| 25 | 200 | 70 - 75 | 111 | 230,4 | 6,3 - 6,8 | 0 | 18 | 16 |
| 25 | 180 | 55 - 70 | 111 | 115,2 | 4,5 - 5,1 | 0 | 20 | 10 |
| 30 | 180 | 60 - 70 | 111 | 230,4 | 5,3 - 5,8 | 0 | 21 | 14 |

Beispiel 7

Nach dem beschriebenen Verfahren wurde der Pulverlack mit folgender Rezeptur hergestellt, appliziert und zwischen 200 °C und 180 °C eingebrannt.

480,0 Gew.-T. Epoxid II.1
70,0 Gew.-T. Vernetzer gemäß I.4
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60 °∡ | MEK-Festigkeit |
| 12 | 200 | 55 - 60 | 125 | 115,2 | 6,8 - 7,2 | 0 | 19 | 12 |
| 15 | 200 | 50 - 65 | 125 | 115,2 | 6,3 - 6,6 | 0 | 18 | 18 |
| 20 | 200 | 65 - 70 | 125 | 345,6 | 6,0 - 6,6 | 0 | 19 | 26 |
| 20 | 180 | 60 - 70 | 111 | 115,2 | 5,1 - 5,2 | 0 | 20 | 10 |
| 30 | 180 | 60 | 125 | 230,4 | 5,0 - 5,5 | 0 | 19 | 18 |

Beispiel 8

Nach dem beschriebenen Verfahren wurde der Pulverlack mit folgender Rezeptur hergestellt, appliziert und zwischen 200 °C und 180 °C eingebrannt.

470,0 Gew.-T. Epoxid gemäß II.1
80,0 Gew.-T. Vernetzer gemäß I.4
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60 °∡ | MEK-Festigkeit |
| 12 | 200 | 60 | 111 | 230,4 | 6,3 - 7,9 | 0 | 25 | 24 |
| 15 | 200 | 50 - 55 | 125 | 115,2 | 7,6 - 8.7 | 0 | 24 | 30 |
| 20 | 200 | 65 | 125 | 345,6 | 7,0 - 7,6 | 0 | 26 | 36 |
| 20 | 180 | 60 - 70 | 111 | 115,2 | 5,6 - 5,9 | 0 | 26 | 18 |
| 30 | 180 | 50 - 60 | 125 | 230,4 | 5,7 - 6,1 | 0 | 25 | 26 |

Beispiel 9

Nach dem beschriebenen Verfahren wurde der Pulverlack mit folgender Rezeptur hergestellt, appliziert und zwischen 200 °C und 180 °C eingebrannt.

480,0 Gew.-T. Epoxid gemäß II.1
70,0 Gew.-T. Vernetzer gemäß I.5
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60 °∡ | MEK-Festigkeit |
| 20 | 200 | 50 - 60 | 100 | 115,2 | 5,2 - 5,6 | 0 | 19 | 10 |
| 25 | 200 | 60 | 100 | 115,2 | 5,9 - 6,3 | 0 | 21 | 12 |
| 30 | 200 | 55 - 60 | 100 | 230,4 | 6,0 - 6,2 | 0 | 20 | 14 |
| 30 | 180 | 60 - 65 | 100 | 115,2 | 4,1 - 4,9 | 0 | 22 | 10 |

Beispiel 10

Nach dem beschriebenen Verfahren wurde der Pulverlack mit folgender Rezeptur hergestellt, appliziert und zwischen 200 °C und 180 °C eingebrannt.

220,0 Gew.-T. Epoxid gemäß II.1

260,0 Gew.-T. Epoxid gemäß II.2
70,0 Gew.-T. Vernetzer gemäß I.5
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60°∡ | MEK-Festigkeit |
| 12 | | 60 - 70 | 111 | 230,4 | 5,1 - 5,7 | 0 | 19 | 70 |
| 15 | 200 | 60 | 100 | 345,6 | 5,9 - 6,4 | 0 | 21 | 80 |
| 20 | | 60 - 65 | 111 | 460,8 | 6,3 - 6,9 | 0 | 20 | 88 |
| 15 | | 55 - 65 | 100 | 115,2 | 3,6 - 4,0 | 0 | 18 | 56 |
| | 180 | | | | | | | |
| 20 | | 60 - 70 | 111 | 115,2 | 4,3 - 4,7 | 0 | 20 | 64 |

Beispiel 11

Nach dem beschriebenen Verfahren wurde der Pulverlack mit folgender Rezeptur hergestellt, appliziert und zwischen 180 °C und 220 °C eingebrannt.
490,0 Gew.-T. Epoxid II.1
60,0 Gew.-T. Vernetzer gemäß I.8
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60°∡ | MEK-Festigkeit |
| 10 | 220 | 50 - 60 | 100 | 115,2 | 3,8 - 4,2 | 0 | 10 | 24 |
| 20 | | 45 - 60 | 111 | <115,2 | 3,1 | 0 | 9 | 24 |
| 25 | 200 | 60 | 111 | <115,2 | 3,2 - 3,4 | 0 | 9 | 26 |
| 30 | | 50 - 60 | 100 | 115,2 | 4,0 - 5,7 | 0 | 10 | 30 |
| 30 | 180 | 60 | 100 | <115,2 | 3,1 - 3,5 | 0 | 9 | 24 |

Beispiel 12

Nach dem beschriebenen Verfahren wurde der Pulverlack mit folgender Rezeptur hergestellt, appliziert und zwischen 180 °C und 220 °C eingebrannt.
485,0 Gew.-T. Epoxid gemäß II.1
65,0 Gew.-T. Vernetzer gemäß I.8
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit/Temp. °C | | SD | HB | Imp. rev. | ET | GS | GG 60°∡ | MEK-Festigkeit |
| 10 | 220 | 60 - 70 | 125 | 115,2 | 4,7 - 5,6 | 0 | 14 | 24 |
| 20 | 200 | 40 - 60 | 125 | 115,2 | 5,8 - 6,1 | 0 | 13 | 28 |
| 25 | | 60 - 80 | 125 | 115,2 | 5,7 - 6,4 | 0 | 15 | 32 |
| 30 | | 60 - 70 | 125 | 230,4 | 5,8 - 6,5 | 0 | 13 | 36 |
| 25 | 180 | 60 - 75 | 125 | <115,2 | 4,5 - 5,8 | 0 | 13 | 20 |
| 30 | | 40 - 50 | 125 | 115,2 | 6,0 - 6,6 | 0 | 15 | 26 |

Beispiel 13

Nach dem beschriebenen Verfahren wurde der Pulverlack mit folgender Rezeptur hergestellt, appliziert und zwischen 180 °C und 220 °C eingebrannt.

480,0 Gew.-T. Epoxid gemäß II.1
70,0 Gew.-T. Vernetzer gemäß I.8
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60°∡ | MEK-Festigkeit |
| 10 | 220 | 60 - 70 | 111 | 115,2 | 4,0 - 4,5 | 0 | 21 | 40 |
| 15 | 200 | 50 - 60 | 111 | 115,2 | 4,2 - 5,7 | 0 | 22 | 38 |
| 20 | | 60 | 125 | 115,2 | 4,9 - 5,7 | 0 | 24 | 44 |
| 25 | | 60 - 85 | 125 | 230,4 | 5,0 - 5,9 | 0 | 24 | 50 |
| 25 | 180 | 60 | 111 | <115,2 | 4,1 - 4,2 | 0 | 21 | 28 |
| 30 | | 60 - 70 | 111 | 115,2 | 4,6 - 4,9 | 0 | 21 | 30 |

Beispiel 14

Nach dem beschriebenen Verfahren wurde der Pulverlack mit folgender Rezeptur hergestellt, appliziert und zwischen 170 °C und 220 °C eingebrannt.

475,0 Gew.-T. Epoxid gemäß II.1
75,0 Gew.-T. Vernetzer gemäß I.8
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60°∡ | MEK-Festigkeit |
| 10 | 220 | 60 - 75 | 111 | 115,2 | 5,1 - 5,5 | 0 | 32 | 70 |
| 15 | 200 | 60 - 65 | 111 | 115,2 | 5,2 - 5,7 | 0 | 32 | 70 |
| 20 | | 60 - 70 | 111 | 115,2 | 6,2 - 6,6 | 0 | 31 | 80 |
| 25 | | 60 - 75 | 111 | 230,4 | 5,8 - 6,0 | 0 | 32 | 90 |
| 20 | 180 | 50 - 80 | 111 | 115,2 | 5,2 - 6,3 | 0 | 32 | 70 |
| 25 | | 70 - 90 | 111 | 115,2 | 4,2 - 5,6 | 0 | 28 | 80 |
| 25 | 170 | 50 - 70 | 111 | <115,2 | 4,2 - 4,6 | 0 | 26 | 60 |

Beispiel 15

Nach dem beschriebenen Verfahren wurde der Pulverlack mit folgender Rezeptur hergestellt, appliziert und bei 200 °C eingebrannt.

480,0 Gew.-T. Epoxid II.1
70,0 Gew.-T. Vernetzer gemäß I.9
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60°∡ | MEK-Festigkeit |
| 12 | 200 | 65 - 70 | 111 | <115,2 | 2,5 - 3,1 | 0 | 14 | 10 |
| 15 | | 60 - 65 | 111 | 115,2 | 3,9 - 4,3 | 0 | 15 | 16 |
| 20 | | 65 - 70 | 111 | 230,4 | 4,1 - 4,8 | 0 | 14 | 24 |

Beispiel 16

Nach dem beschriebenen Verfahren wurde der Pulverlack mit folgender Rezeptur hergestellt, appliziert und zwischen 200 °C und 180 °C eingebrannt.

480,0 Gew.-T. Epoxid gemäß II.1
70,0 Gew.-T. Vernetzer gemäß I.10
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60°∡ | MEK-Festigkeit |
| 15 | 200 | 50 - 60 | 111 | 115,2 | 5,3 - 6,5 | 0 | 19 | 8 |
| 20 | | 60 - 70 | 111 | 115,2 | 7,3 - 7,5 | 0 | 20 | 10 |
| 25 | | 65 - 70 | 111 | 230,4 | 7,4 - 7,7 | 0 | 21 | 16 |
| 30 | 180 | 45 - 55 | 111 | 115,2 | 4,5 - 5,5 | 0 | 20 | 10 |

Beispiel 17

Nach dem beschriebenen Verfahren wurde der Pulverlack mit folgender Rezeptur hergestellt, appliziert und zwischen 200 °C und 180 °C eingebrannt.

480,0 Gew.-T. Epoxid gemäß II.1

70,0 Gew.-T. Vernetzer gemäß I.15
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60°∡ | MEK-Festigkeit |
| 10 | 200 | 60 - 70 | 125 | 115,2 | 4,0 - 4,2 | 0 | 17 | 50 |
| 15 | | 40 - 45 | 100 | 345,6 | 7,3 - 7,6 | 0 | 16 | 120 |
| 20 | | 50 - 60 | 111 | 230,4 | 7,2 - 7,3 | 0 | 15 | 140 |
| 20 | 180 | 70 - 90 | 111 | 115,2 | 3,9 - 5,0 | 0 | 20 | 22 |
| 25 | | 50 - 60 | 100 | 115,2 | 4,5 - 4,8 | 0 | 20 | 46 |

Beispiel 18

Nach dem beschriebenen Verfahren wurde der Pulverlack mit folgender Rezeptur hergestellt, appliziert und zwischen 200 °C und 180 °C eingebrannt.
490,0 Gew.-T. Epoxid II.1
60,0 Gew.-T. Vernetzer gemäß I.15
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60°∡ | MEK-Festigkeit |
| 15 | 200 | 50 - 60 | 111 | 115,2 | 4,1 - 4,4 | 0 | 20 | 40 |
| 20 | | 60 - 70 | 100 | 230,4 | 6,0 - 6,4 | 0 | 18 | 80 |
| 25 | | 50 - 60 | 111 | 230,4 | 6,3 - 6,9 | 0 | 20 | 110 |
| 25 | 180 | 55 - 65 | 111 | 115,2 | 4,0 - 4,8 | 0 | 21 | 18 |
| 30 | | 50 - 60 | 100 | 115,2 | 4,7 - 5,1 | 0 | 19 | 48 |

Beispiel 19

Nach dem beschriebenen Verfahren wurde der Pulverlack mit folgender Rezeptur hergestellt, appliziert und zwischen 180 °C und 220 °C eingebrannt.
485,0 Gew.-T. Epoxid gemäß II.1
65,0 Gew.-T. Vernetzer gemäß I.16
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60°∡ | MEK-Festigkeit |
| 10 | 220 | 50 - 60 | 100 | 115,2 | 3,5 - 4,1 | 0 | 11 | 26 |
| 15 | 200 | 45 - 55 | 100 | <115,2 | 3,8 - 4,0 | 0 | 10 | 28 |
| 20 | | 45 - 60 | 100 | 115,2 | 3,5 - 3,7 | 0 | 10 | 28 |
| 25 | | 60 - 70 | 111 | 115,2 | 3,8 - 4,5 | 0 | 10 | 30 |
| 30 | 180 | 45 - 60 | 111 | <115,2 | 3,0 - 3,9 | 0 | 13 | 22 |

Beispiel 20

Nach dem beschriebenen Verfahren wurde der Pulverlack mit folgender Rezeptur hergestellt, appliziert und bei 200 °C eingebrannt.

480,0 Gew.-T. Epoxid gemäß II.1
70,0 Gew.-T. Vernetzer gemäß I.17
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60°∡ | MEK-Festigkeit |
| 15 | 200 | 50 - 55 | 111 | 230,4 | 7,0 | 0 | 18 | 100 |
| 20 | | 55 - 60 | 111 | 230,4 | 6,5 - 6,8 | 0 | 18 | 140 |

Beispiel 21

Nach dem beschriebenen Verfahren wurde der Pulverlack mit folgender Rezeptur hergestellt, appliziert und zwischen 200 °C und 180 °C eingebrannt.

490,0 Gew.-T. Epoxid gemäß II.1
60,0 Gew.-T. Vernetzer gemäß I.17
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60°∡ | MEK-Festigkeit |
| 15 | 200 | 50 - 60 | 111 | 115,2 | 5,9 - 6,5 | 0 | 16 | 40 |
| 20 | | 55 - 65 | 111 | 230,4 | 6,3 - 6,9 | 0 | 14 | 82 |
| 30 | 180 | 50 - 55 | 100 | 115,2 | 4,9 - 5,5 | 0 | 15 | 56 |

Beispiel 22

Nach dem beschriebenen Verfahren wurde der Pulverlack mit folgender Rezeptur hergestellt, appliziert und zwischen 180 °C und 200 °C eingebrannt.

475,0 Gew.-T. Epoxid II.1
75,0 Gew.-T. Vernetzer gemäß I.18
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60°∡ | MEK-Festigkeit |
| 15 | | 60 | 100 | 460,8 | 3,4 - 4,0 | 0 | 7 | 22 |
| 20 | 200 | 45 - 60 | 100 | 460,8 | 5,0 - 5,2 | 0 | 7 | 40 |
| 25 | | 50 - 60 | 100 | 460,8 | 5,3 - 5,6 | 0 | 7 | 50 |
| 25 | 180 | 55 - 65 | 100 | 115,2 | 3,3 - 3,4 | 0 | 11 | 10 |
| 30 | | 50 - 60 | 100 | 115,2 | 4,4 - 4,6 | 0 | 12 | 20 |

Beispiel 23

Nach dem beschriebenen Verfahren wurde der Pulverlack mit folgender Rezeptur hergestellt, appliziert und zwischen 200 °C und 180 °C eingebrannt.

260,0 Gew.-T. Epoxid gemäß II.2
220,0 Gew.-T. Epoxid gemäß II.1
70,0 Gew.-T. Vernetzer gemäß I.18
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60°∡ | MEK-Festigkeit |
| 15 | 200 | 50 - 60 | 100 | 230,4 | 3,7 - 4,1 | 0 | 9 | 20 |
| 20 | | 55 - 70 | 111 | 230,4 | 4,9 - 5,5 | 0 | 10 | 28 |
| 25 | | 60 - 70 | 100 | 345,6 | 5,2 - 5,6 | 0 | 9 | 46 |
| 25 | 180 | 60 | 100 | 115,2 | 3,2 - 3,6 | 0 | 12 | 10 |
| 30 | | 60 - 70 | 100 | 115,2 | 4,3 - 4,8 | 0 | 12 | 18 |

Beispiel 24

Nach dem beschriebenen Verfahren wurde der Pulverlack mit folgender Rezeptur hergestellt, appliziert und zwischen 180 °C und 200 °C eingebrannt.

485,0 Gew.-T. Epoxid gemäß II.1
65,0 Gew.-T. Vernetzer gemäß I.18
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60°∡ | MEK-Festigkeit |
| 20 | 200 | 50 - 55 | 100 | 115,2 | 3,1 - 4,0 | 0 | 6 | 20 |
| 25 | | 50 - 65 | 100 | 230,4 | 5,2 | 0 | 6 | 24 |
| 30 | | 50 - 60 | 100 | 691,2 | 5,3 - 6,0 | 0 | 6 | 26 |
| 25 | 180 | 50 - 70 | 100 | <115,2 | 3,6 - 4,0 | 0 | 12 | 8 |
| 30 | | 50 - 55 | 100 | 115,2 | 4,5 - 5,4 | 0 | 12 | 14 |

Beispiel 25

Nach dem beschriebenen Verfahren wurde der Pulverlack mit folgender Rezeptur hergestellt, appliziert und zwischen 180 °C und 200 °C eingebrannt.

500,0 Gew.-T. Epoxid gemäß II.1
50,0 Gew.-T. Vernetzer gemäß I.18
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60° ∡ | MEK-Festigkeit |
| 20<br>25<br>30 | 200 | 40 - 50<br>50 - 65<br>40 | 100<br>100<br>100 | <115,2<br>115,2<br>115,2 | 2,8 - 3,8<br>5,1 - 5,5<br>6,3 - 6,6 | 0<br>0<br>0 | 5<br>6<br>6 | 12<br>18<br>20 |
| 20<br>30 | 180 | 50 - 60<br>50 - 60 | 100<br>100 | <115,2<br>115,2 | 4,0<br>4,4 - 4,6 | 0<br>0 | 12<br>12 | 8<br>10 |

Beispiel 26

Nach dem beschriebenen Verfahren wurde der Pulverlack mit folgender Rezeptur hergestellt, appliziert und zwischen 200 °C und 180 °C eingebrannt.

480,0 Gew.-T. Epoxid II.1
70,0 Gew.-T. Vernetzer gemäß I.19
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60° ∡ | MEK-Festigkeit |
| 15<br>20<br>25 | 200 | 50 - 60<br>50 - 60<br>60 - 70 | 111<br>111<br>111 | 115,2<br>230,4<br>230,4 | 5,5 - 5,9<br>5,7 - 6,3<br>6,0 - 6,7 | 0<br>0<br>0 | 21<br>20<br>23 | 80<br>100<br>120 |
| 30 | 180 | 60 - 70 | 100 | 115,2 | 4,7 - 5,1 | 0 | 25 | 60 |

Beispiel 27

Nach dem beschriebenen Verfahren wurde der Pulverlack mit folgender Rezeptur hergestellt, appliziert und zwischen 170 °C und 220 °C eingebrannt.

475,0 Gew.-T. Epoxid gemäß II.1
75,0 Gew.-T. Vernetzer gemäß I.21
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60° ∡ | MEK-Festigkeit |
| 10 | 220 | 45 - 55 | 100 | 115,2 | 5,0 - 5,3 | 0 | 9 | 80 |
| 15<br>20<br>25 | 200 | 50<br>40 - 50<br>50 - 65 | 100<br>111<br>100 | 115,2<br>230,4<br>345,6 | 6,0 - 6,2<br>6,8<br>5,8 - 5,9 | 0<br>0<br>0 | 8<br>8<br>8 | 80<br>90<br>90 |
| 20<br>25 | 180 | 65<br>60 - 70 | 100<br>100 | <115,2<br>115,2 | 4,1 - 4,4<br>4,7 - 4,9 | 0<br>0 | 11<br>10 | 70<br>80 |
| 30 | 170 | 50 - 60 | 100 | <115,2 | 3,5 - 4,1 | 0 | 10 | 60 |

Beispiel 28

Nach dem beschriebenen Verfahren wurde der Pulverlack mit folgender Rezeptur hergestellt, appliziert und zwischen 180 °C und 200 °C eingebrannt.

485,0 Gew.-T. Epoxid gemäß II.1
65,0 Gew.-T. Vernetzer gemäß I.21
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60°∡ | MEK-Festigkeit |
| 20 | 200 | 45 - 60 | 100 | 230,4 | 5,1 - 6,1 | 0 | 10 | 24 |
| 25 | 200 | 50 - 70 | 125 | 230,4 | 5,4 - 6,0 | 0 | 10 | 28 |
| 30 | 200 | 70 | 125 | 345,6 | 5,3 - 6,2 | 0 | 10 | 30 |
| 25 | 180 | 60 - 80 | 100 | 115,2 | 3,5 - 4,2 | 0 | 12 | 20 |
| 30 | 180 | 60 | 100 | 115,2 | 4,6 - 5,0 | 0 | 12 | 28 |

Beispiel 29

Nach dem beschriebenen Verfahren wurde der Pulverlack mit folgender Rezeptur hergestellt, appliziert und zwischen 200 °C und 170 °C eingebrannt.

215,0 Gew.-T. Epoxid gemäß II.1
260,0 Gew.-T. Epoxid gemäß II.2
75,0 Gew.-T. Vernetzer gemäß I.21
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60°∡ | MEK-Festigkeit |
| 15 | 200 | 60 - 70 | 111 | 115,2 | 5,9 - 6,3 | 0 | 9 | 76 |
| 20 | 200 | 55 - 70 | 111 | 230,4 | 6,3 - 6,5 | 0 | 10 | 88 |
| 25 | 200 | 50 - 60 | 100 | 230,4 | 6,0 - 6,7 | 0 | 9 | 92 |
| 20 | 180 | 50 - 60 | 111 | <115,2 | 3,9 - 4,4 | 0 | 10 | 66 |
| 25 | 180 | 60 | 111 | 115,2 | 4,2 - 4,8 | 0 | 11 | 78 |
| 30 | 170 | 50 - 60 | 100 | <115,2 | 3,4 - 3,9 | 0 | 10 | 62 |

Beispiel 30

Nach dem beschriebenen Verfahren wurde der Pulverlack mit folgender Rezeptur hergestellt, appliziert und bei 200 °C eingebrannt.

480,0 Gew.-T. Epoxid II.1
70,0 Gew.-T. Vernetzer gemäß I.23
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60°∡ | MEK-Festigkeit |
| 12 | 200 | 60 | 111 | <115,2 | 6,0 - 6,4 | 0 | 17 | 6 |
| 15 | | 45 - 55 | 111 | 115,2 | 5,3 - 6,5 | 0 | 19 | 10 |
| 20 | | 60 - 70 | 111 | 230,4 | 7,4 - 7,5 | 0 | 20 | 16 |

Beispiel 31

Nach dem beschriebenen Verfahren wurde der Pulverlack mit folgender Rezeptur hergestellt, appliziert und zwischen 200 °C und 180 °C eingebrannt.

500,0 Gew.-T. Epoxid gemäß II.1
50,0 Gew.-T. Vernetzer gemäß I.25
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60°∡ | MEK-Festigkeit |
| 15 | 200 | 50 - 60 | 111 | 115,2 | 5,3 - 5,8 | 0 | 34 | 20 |
| 20 | | 50 - 65 | 111 | 115,2 | 5,6 - 6,2 | 0 | 36 | 40 |
| 25 | | 55 - 65 | 125 | 230,4 | 6,3 - 6,7 | 0 | 35 | 60 |
| 30 | 180 | 60 | 111 | 115,2 | 5,0 - 5,5 | 0 | 36 | 30 |

Beispiel 32

Nach dem beschriebenen Verfahren wurde der Pulverlack mit folgender Rezeptur hergestellt, appliziert und bei zwischen 200 °C und 180 °C eingebrannt.

490,0 Gew.-T. Epoxid II.1
60,0 Gew.-T. Vernetzer gemäß I.25
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60°∡ | MEK-Festigkeit |
| 15 | 200 | 50 | 125 | 115,2 | 6,4 - 6,5 | 0 | 37 | 20 |
| 20 | | 55 - 60 | 125 | 230,4 | 6,4 - 6,9 | 0 | 37 | 80 |
| 25 | | 50 - 60 | 125 | 230,4 | 7,6 - 8,8 | 0 | 36 | 110 |
| 25 | 180 | 50 - 65 | 111 | 115,2 | 5,2 - 5,7 | 0 | 34 | 30 |
| 30 | | 45 - 55 | 125 | 115,2 | 6,0 - 6,3 | 0 | 36 | 60 |

Beispiel 33

Nach dem beschriebenen Verfahren wurde der Pulverlack mit folgender Rezeptur hergestellt, appliziert und zwischen 200 °C und 180 °C eingebrannt.

480,0 Gew.-T. Epoxid gemäß II.1
70,0 Gew.-T. Vernetzer gemäß I.25
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60°∡ | MEK-Festigkeit |
| 15 | | 60 | 125 | 115,2 | 5,5 - 5,8 | 0 | 38 | 20 |
| 20 | 200 | 50 - 65 | 125 | 230,4 | 6,2 - 6,6 | 0 | 41 | 100 |
| 25 | | 50 - 60 | 125 | 345,6 | 6,7 - 7,6 | 0 | 41 | >200 |
| 25 | 180 | 55 - 60 | 125 | 115,2 | 4,8 - 5,2 | 0 | 40 | 30 |
| 30 | | 50 - 60 | 125 | 230,4 | 5,5 - 5,9 | 0 | 42 | 70 |

**IV. Carboxylgruppenhaltiger Polyester**

Zur Herstellung von Hybrid-Pulverlacken wurde der nachfolgend beschriebene carboxylgruppenhaltige Polyester mit folgenden Kenndaten (Angaben des Herstellers) eingesetzt:

| | |
|---|---|
| Säurezahl | 52 - 58 mg KOH/g |
| Schmelzbereich | 104 - 106 °C |
| Glasumwandlungstemperatur | ca. 58 °C |
| Viskosität bei 185 °C | 33400 mPa•s |

**V. Hybrid-Pulverlacke**

Die Aufbereitung der Rohstoffe sowie die Herstellung und Applikation erfolgt analog III.

Beispiel 1

350,0 Gew.-T. Epoxid gemäß II.1
145,0 Gew.-T. Polyester gemäß IV
55,0 Gew.-T. Vernetzer gemäß I.1
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60°∡ | MEK-Festigkeit |
| 10 | | 80 | 111 | 115,2 | 5,7 - 6,5 | 0 | 51 | 24 |
| 15 | 200 | 70 - 80 | 111 | 115,2 | 6,2 - 6,6 | 0 | 51 | 32 |
| 20 | | 60 - 70 | 111 | 115,2 | 5,7 - 6,8 | 0 | 50 | 36 |
| 20 | 180 | 70 - 80 | 125 | 115,2 | 4,7 - 5,6 | 0 | 51 | 22 |
| 30 | | 50 - 60 | 125 | 115,2 | 5,5 - 6,8 | 0 | 48 | 28 |
| 30 | 170 | 50 - 60 | 111 | 115,2 | 5,0 - 5,6 | 0 | 48 | 10 |

Beispiel 2

Nach dem beschriebenen Verfahren wurde der Pulverlack mit folgender Rezeptur hergestellt, appliziert und zwischen 180 °C und 200 °C eingebrannt.

390,0 Gew.-T. Epoxid gemäß II.1
75,0 Gew.-T. Vernetzer gemäß I.1
400,0 Gew.-T. Weißpigment ($TiO_2$)
50,0 Gew.-T. Verlaufmittel-Masterbatch
85,0 Gew.-T. Polyester gemäß IV

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60°∡ | MEK-Festigkeit |
| 10 | 200 | 65 - 80 | 125 | 115,2 | 3,8 - 4,2 | 0 | 43 | 28 |
| 15 | 200 | 60 - 65 | 125 | 115,2 | 5,6 - 5,7 | 0 | 43 | 46 |
| 20 | 200 | 60 | 125 | 115,2 | 4,9 - 5,3 | 0 | 40 | 54 |
| 20 | 180 | 70 - 80 | 125 | 115,2 | 3,7 - 4,2 | 0 | 47 | 20 |
| 30 | 180 | 60 - 70 | 125 | 115,2 | 4,0 - 4,5 | 0 | 49 | 28 |

Beispiel 3

Nach dem beschriebenen Verfahren wurde der Pulverlack mit folgender Rezeptur hergestellt, appliziert und zwischen 200 °C und 180 °C eingebrannt.

145,0 Gew.-T. Polyester gemäß IV
350,0 Gew.-T. Epoxid II.1
55,0 Gew.-T. Vernetzer gemäß I.11
400,0 Gew.-T. Weißpigment ($TiO_2$)
50,0 Gew.-T. Verlaufmittel-Masterbatch

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60°∡ | MEK-Festigkeit |
| 12 | 200 | 60 - 70 | 111 | 115,2 | 5,3 - 5,7 | 0 | 45 | 20 |
| 15 | 200 | 60 | 125 | 115,2 | 5,6 - 6,4 | 0 | 48 | 24 |
| 20 | 200 | 60 - 75 | 111 | 230,4 | 5,9 - 6,6 | 0 | 50 | 28 |
| 20 | 180 | 50 - 70 | 111 | 115,2 | 4,3 - 5,0 | 0 | 46 | 18 |
| 30 | 180 | 60 - 70 | 125 | 115,2 | 4,7 - 5,5 | 0 | 48 | 22 |

Beispiel 4

Nach dem beschriebenen Verfahren wurde der Pulverlack mit folgender Rezeptur hergestellt, appliziert und zwischen 200 °C und 180 °C eingebrannt.

390,0 Gew.-T. Epoxid gemäß II.1
75,0 Gew.-T. Vernetzer gemäß I.16
400,0 Gew.-T. Weißpigment ($TiO_2$)
50,0 Gew.-T. Verlaufmittel-Masterbatch
85,0 Gew.-T. Polyester gemäß IV

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60°∡ | MEK-Festigkeit |
| 15 | 200 | 60 - 65 | 100 | 115,2 | 5,8 - 6,2 | 0 | 34 | 20 |
| 20 | 200 | 60 | 111 | 230,4 | 5,2 - 6,0 | 0 | 36 | 26 |
| 25 | 200 | 60 - 70 | 100 | 345,6 | 6,9 - 7,5 | 0 | 32 | 28 |
| 30 | 180 | 50 - 60 | 100 | 230,4 | 3,7 - 4,2 | 0 | 40 | 14 |

Beispiel 5

Nach dem beschriebenen Verfahren wurde der Pulverlack mit folgender Rezeptur hergestellt, appliziert und zwischen 180 °C und 200 °C eingebrannt.

390,0 Gew.-T. Epoxid gemäß II.1
75,0 Gew.-T. Vernetzer gemäß I.18
400,0 Gew.-T. Weißpigment ($TiO_2$)
50,0 Gew.-T. Verlaufmittel-Masterbatch
85,0 Gew.-T. Polyester gemäß IV

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60°∡ | MEK-Festigkeit |
| 10 | 200 | 50 - 60 | 111 | 345,6 | 6,0 | 0 | 23 | 20 |
| 15 | 200 | 80 - 90 | 125 | 230,4 | 5,3 - 6,1 | 0 | 19 | 24 |
| 20 | 200 | 50 - 60 | 125 | 691,2 | 7,1 - 7,3 | 0 | 19 | 30 |
| 20 | 180 | 70 - 75 | 111 | 115,2 | 2,5 - 2,9 | 0 | 22 | 10 |
| 30 | 180 | 50 - 70 | 125 | 230,4 | 3,5 - 4,0 | 0 | 20 | 14 |

Beispiel 6

Nach dem beschriebenen Verfahren wurde der Pulverlack mit folgender Rezeptur hergestellt, appliziert und zwischen 180 °C und 200 °C eingebrannt.

390,0 Gew.-T. Epoxid gemäß II.1
75,0 Gew.-T. Vernetzer gemäß I.21
400,0 Gew.-T. Weißpigment ($TiO_2$)
50,0 Gew.-T. Verlaufmittel-Masterbatch
85,0 Gew.-T. Polyester gemäß IV

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60°∡ | MEK-Festigkeit |
| 10 | 200 | 80 - 100 | 111 | <115,2 | 4,0 | 0 | 11 | 50 |
| 15 | 200 | 70 - 80 | 111 | 115,2 | 5,3 - 5,6 | 0 | 15 | 80 |
| 20 | 200 | 60 - 70 | 111 | 230,4 | 5,3 - 5,9 | 0 | 12 | 110 |
| 20 | 180 | 80 - 100 | 100 | <115,2 | 3,2 - 3,4 | 0 | 11 | 30 |
| 30 | 180 | 70 - 80 | 111 | 115,2 | 4,1 - 4,4 | 0 | 11 | 40 |

Beispiel 7

Nach dem beschriebenen Verfahren wurde der Pulverlack mit folgender Rezeptur hergestellt, appliziert und zwischen 200 °C und 180 °C eingebrannt.

380,0 Gew.-T. Epoxid II.1
65,0 Gew.-T. Vernetzer gemäß I.25
400,0 Gew.-T. Weißpigment ($TiO_2$)
50,0 Gew.-T. Verlaufmittel-Masterbatch
105,0 Gew.-T. Polyester gemäß IV

| Einbrennbedingungen | | Mechanische Kenndaten | | | | | | Lösungsmittelresistenz |
|---|---|---|---|---|---|---|---|---|
| Zeit min/Temp. °C | | SD | HB | Imp. rev. | ET | GS | GG 60 °∡ | MEK-Festigkeit |
| 15 | | 50 - 60 | 111 | <115,2 | 4,3 - 4,5 | 0 | 34 | 20 |
| 20 | 200 | 60 | 100 | 115,2 | 4,7 - 5,1 | 0 | 38 | 30 |
| 25 | | 55 - 65 | 111 | 230,4 | 5,3 - 5,8 | 0 | 37 | 46 |
| 30 | 180 | 50 - 60 | 100 | <115,2 | 4,4 - 4,9 | 0 | 40 | 34 |

**Patentansprüche**

1. Salze der Pyromellitsäure,
dadurch gekennzeichnet,
daß ihre Amin-Komponente folgende Zusammensetzung hat:

A) 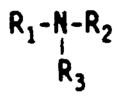 oder B) 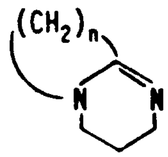

wobei $R_1$, $R_2$, $R_3$ gleiche oder verschiedene aliphatische, cycloaliphatische, araliphatische, aromatische Kohlenwasserstoffreste mit 1 - 20 C-Atomen, wobei in der C-Kette eine oder mehrere $CH_2$-Gruppen durch O-Atome, durch $NR_4$ mit $R_4$ = $C_{1-6}$-Alkyl, durch CH-OH-Gruppen, und/oder eine oder mehrere endständige Methylgruppen durch dialkylsubstituierte Aminogruppen mit 1 bis 6 Kohlenstoffatomen, ersetzt sein können und $R_1$ und $R_2$ einen gemeinsamen Ring bilden können, in dem eine $CH_2$-Gruppe durch ein O-Atom oder durch eine $NR_4$-Gruppe ersetzt sein kann, und $R_1$ = $R_2$ = $R_3$ = $-CH_2-CH_2-$ über ein gemeinsames N-Atom gebunden sind, und n : 3 - 11 bedeuten.

2. Verfahren zur Herstellung von Salzen der Pyromellitsäure nach Anspruch 1,
dadurch gekennzeichnet,
daß 1 mol Pyromellitsäure mit 0,5 - 2 mol Amin A) oder B) in $H_2O$ bzw. Ethanol bei 50 - 100 °C umgesetzt und nach beendeter Reaktion das Reaktionsprodukt vom Lösungsmittel abgetrennt wird.

3. Verwendung der Salze der Pyromellitsäure nach Anspruch 1 zur Herstellung von matten Epoxid-Pulverbeschichtungen, wobei das EP-Harz 1 - 12 Gew.-%, vorzugsweise 2 - 7 Gew-%, insbesondere 3 - 5,5 Gew.-% der Salze enthält.

4. Verwendung der Salze der Pyromellitsäure nach Anspruch 1 zur Herstellung von matten Hybrid-Pulverbeschichtungen, wobei das EP-Harz neben COOH-Gruppen enthaltenden Polyestern und gegebenenfalls zusätzlich vorhandenen blockierten Polyisocyanaten und/oder blockierungsmittelfreien Polyisocyanaten 1 - 12 Gew.-%, vorzugsweise 2 - 7 Gew.-%, der Salze enthält.